# FASCICULE DE BREVET EUROPEEN

(11) **EP 1 333 022 B1**
(45) Date de publication et mention de la délivrance du brevet: **07.12.2011**
(21) Numéro de dépôt: 03290080.5
(22) Date de dépôt: 13.01.2003
(51) Int. Cl.: A61K 8/36, A61K 8/37

(54) **Compositions comprenant des dérivés du cyclopentane, et utilisation pour favoriser la desquamation**
Zusammensetzungen enthaltend Cyclopentanderivate und deren Verwendung zur Förderung der Abschuppung der Haut
Compounds containing derivatives of cyclopentane and their use to stimulate skin peeling

(30) Priorité: 04.02.2002 FR 0201280
(43) Date de publication de la demande: 06.08.2003
(73) Titulaire: L'Oréal, 75008 Paris (FR)
(72) Inventeur: Leveque, Jean-Luc, 75017 Paris (FR); Dalko, Maria, 91190 Gif S/Yvette (FR); Boulle, Christophe, 77400 Lagny S/Marne (FR)
(74) Mandataire: Kromer, Christophe

(56) Documents cités:
- EP-A- 0 989 111
- FR-A- 2 718 022
- KIYOTA H ET AL: "Lipase-catalyzed preparation of both enantiomers of methyl jasmonate" TETRAHEDRON: ASYMMETRY, ELSEVIER SCIENCE PUBLISHERS, AMSTERDAM, NL, vol. 12, no. 7, 2001, pages 1035-1038, XP002214851 ISSN: 0957-4166
- DATABASE CA [Online] CHEMICAL ABSTRACTS SERVICE, COLUMBUS, OHIO, US; MIERSCH, OTTO ET AL: "Structure-activity relations of substituted, deleted or stereospecificall altered jasmonic acid in gene expression of barley leaves" XP002215102 extrait de STN Database accession no. 130:264855 & PHYTOCHEMISTRY (1998), VOLUME DATE 1999, 50(3), 353-361, 1999,
- DATABASE CA [Online] CHEMICAL ABSTRACTS SERVICE, COLUMBUS, OHIO, US; WARD, KERRY ET AL: "The induction of proteinase inhibitor II by jasmonates" XP002215103 extrait de STN Database accession no. 127:289066 & PROCEEDINGS OF THE PLANT GROWTH REGULATOR SOCIETY OF AMERICA (1996), 23RD 291-294, 1996,
- DATABASE CA [Online] CHEMICAL ABSTRACTS SERVICE, COLUMBUS, OHIO, US; HERRMANN, G. ET AL: "Biological activity of jasmonic acid conjugates" XP002215105 extrait de STN Database accession no. 112:52370 & CONJUGATED PLANT HORM., PROC. INT. SYMP. (1987), MEETING DATE 1986, 315-22. EDITOR(S): SCHREIBER, K.;SCHUETTE, H. R.; SEMBDNER, G. PUBLISHER: DTSCH. VERLAG WISS., BERLIN, GER. DEM. REP., 1987,
- DATABASE CA [Online] CHEMICAL ABSTRACTS SERVICE, COLUMBUS, OHIO, US; ISHIKAWA, ATSUSHI ET AL: "Structure-activity relationships of jasmonates in the induction of expression of two proteinase inhibitor genes of potato" XP002215104 extrait de STN Database accession no. 121:5244 & BIOSCIENCE, BIOTECHNOLOGY, AND BIOCHEMISTRY (1994), 58(3), 544-7, 1994,
- PATENT ABSTRACTS OF JAPAN vol. 008, no. 011 (C-205), 18 janvier 1984 (1984-01-18) & JP 58 180410 A (SHISEIDO KK; others: 01), 21 octobre 1983 (1983-10-21)
- DATABASE WPI Section Ch, Week 200204 Derwent Publications Ltd., London, GB; Class D21, AN 2002-029066 XP002215117 & JP 2001 207188 A (POLA CHEM IND INC) 31 juillet 2001 (2001-07-31)
- DATABASE CA [Online] CHEMICAL ABSTRACTS SERVICE, COLUMBUS, OHIO, US; ISHIDOYA, TOYOMASA ET AL: "Stress-relieving perfume compositions for activating protease of stratum corneum and normalizing its metabolism" XP002347553 extrait de STN Database accession no. 2001:531908 & JP 2001 199832 A2 (POLA CHEMICAL INDUSTRIES, INC., JAPAN) 24 juillet 2001 (2001-07-24)

## Description

La présente invention concerne l'utilisation de dérivés du cyclopentane pour favoriser la desquamation de la peau et/ou stimuler le renouvellement épidermique.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement de la peau.
Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules mortes situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

La demande JP-A-2001-207188 décrit une composition de parfum contenant du dihydrojasmonate de méthyle.

L'invention a pour but de pallier ces inconvénients de l'art antérieur et de proposer de l'utilisation de composés susceptibles de favoriser la desquamation de la peau et/ou de stimuler le renouvellement épidermique, dont l'utilisation n'entraînerait pas de picotements, de tiraillements, d'échauffements ou de rougeurs désagréables pour l'utilisateur.

Ainsi, l'invention a pour objet l'utilisation cosmétique d'au moins un composé de formule (I) telle que définie ci-après pour favoriser la desquamation de la peau et/ou stimuler le renouvellement épidermique.

Les composés utilisés selon l'invention répondent à la formule (I) suivante : 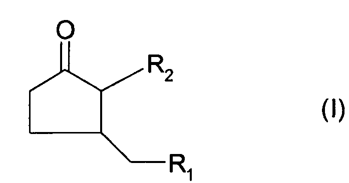 dans laquelle :
- R₁ est un radical choisi parmi -COOR' avec R' désignant un atome d'hydrogène ou un radical hydrocarboné, linéaire, ramifié ou cyclique, saturé ou insaturé, ayant 1 à 18 atomes de carbone ;
- R₂ est un radical hydrocarboné, linéaire, ramifié ou cyclique, comportant au moins une insaturation, ayant 2 à 18 atomes de carbone leurs sels correspondants.

Plus particulièrement, R₁ est choisi parmi les radicaux -COOH, -COOCH₃, - COOC₂H₅, -COOC₃H₇.

R₂ est un radical hydrocarboné, linéaire, ramifié ou cyclique, comportant une double insaturation, ayant 2 à 18 atomes de carbone, notamment 3 à 12 atomes de carbone, et en particulier 3 à 8 atomes de carbone. Plus particulièrement, R₂ représente un radical hydrocarboné linéaire, comportant une seule double insaturation, ayant 2 à 6 atomes de carbone, et notamment un radical -CH₂-CH=CH-C₂H₅.

Les sels des composés utilisables selon l'invention sont en particulier choisis parmi les sels de métal alcalin ou alcalino-terreux, ou encore parmi les sels de zinc, de magnésium ou de strontium, d'une amine organique ou les sels d'ammonium quaternaires.

Les sels des composés conformes à l'invention sont en particulier choisis parmi les sels d'un acide minéral ou organique notamment les chlorhydrates, bromhydrates ou citrates.

Parmi les composés susceptibles d'être employés dans le cadre de l'invention, on peut citer :
- l'acide 3-oxo-2-[(2Z)-2-pentenyl]- cyclopentaneacétique,
- le 3-oxo-2-[(2Z)-2-pentenyl]- cyclopentaneacétate de méthyle.

La quantité de composé de formule (I) utilisable selon l'invention dépend bien évidemment de l'effet recherché et doit être en une quantité efficace pour favoriser la desquamation de la peau et/ou stimuler le renouvellement épidermique. A titre d'exemple, la quantité de composé de formule (I) utilisable selon l'invention peut aller par exemple de 0,01 à 20% et de préférence de 0,5 à 10%, et notamment de 1 à 5% en poids, par rapport au poids total de la composition.

La composition comprenant les composés selon l'invention, seuls ou en mélange, peut comprendre par ailleurs un milieu physiologiquement acceptable, c'est-à-dire un milieu compatible avec toutes les matières kératiniques telles que la peau, le cuir chevelu, les ongles, les muqueuses, les yeux et les cheveux ou toute autre zone cutanée du corps. Cette composition peut être une composition cosmétique ou pharmaceutique et peut donc comprendre un milieu cosmétiquement ou pharmaceutiquement acceptable.

Le milieu physiologiquement acceptable peut comprendre de l'eau, des solvants organiques tels qu'un alcool en C₁-C₈, notamment l'éthanol, l'isopropanol, le tert-butanol, le n-butanol; un polyol tel que la glycérine ; un glycol comme le butylène glycol, l'isoprène glycol, le propylène glycol, les polyéthylène glycols tels que le PEG-8 ; les éthers de polyol.

La composition peut également comprendre une phase grasse, qui peut comprendre des huiles, des gommes, des cires usuellement utilisées dans le domaine d'application considéré. Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

La composition peut contenir également des adjuvants habituels dans le domaine considéré, tels que les tensioactifs, les émulsionnants, les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes, les actifs cosmétiques ou pharmaceutiques. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme tensioactifs susceptibles d'être utilisés, on peut citer par exemple le stéarate de glycérol, le polysorbate 60 et le mélange de PEG-6/PEG-32/Glycol Stéarate vendu sous la dénomination de TefoseR 63 par la société Gattefosse.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Parmi les actifs hydrophiles, on peut citer les protéines ou les hydrolysats de protéine, les acides aminés, les polyols, l'urée, l'allantoïne, les sucres et les dérivés de sucre, les vitamines hydrosolubles, les extraits végétaux et les hydroxyacides.
Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un composé de formule (I) à d'autres agents actifs, tels que :
- les agents améliorant la repousse et/ou sur le ralentissement de la chute des cheveux, comme par exemple les esters d'acide nicotinique, dont notamment le nicotinate de tocophérol, le nicotinate de benzyle et les nicotinates d'alkyles en C1-C6 comme les nicotinates de méthyle ou d'hexyle, les dérivés de pyrimidine, comme le 2,4-diamino 6-piperidinopyrimidine 3-oxyde ou "Minoxidil", les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- lès agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

La composition peut se présenter sous toutes les formes galéniques envisageables.
Notamment, la composition peut avoir la forme de solution aqueuse, alcoolique, hydroalcoolique ou huileuse; de dispersion du type lotion ou sérum; d'émulsion eau-dans-huile, huile-dans-eau ou multiple; de suspension; de microcapsules ou microparticules; de dispersions vésiculaires de type ionique et/ou non ionique; de lotion aqueuse, huileuse ou sous forme de sérum; de capsules, de granulés, de sirops, de comprimés; de mousse, de préparation solide; de composition pour aérosol comprenant également un agent propulseur sous pression.

La composition selon l'invention peut se présenter sous la forme d'une composition pour soins capillaires, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.
Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur; une composition pour traiter certaines maladies de la peau comme l'eczéma, la rosacée, le psoriasis, les lichens, les prurits sévères.

La composition selon l'invention trouve une application toute particulière comme composition cosmétique ou pharmaceutique destinée au soin de la peau du visage, du corps ou du cuir chevelu, notamment pour favoriser la desquamation de la peau, stimuler le renouvellement épidermique.

L'invention est illustrée plus en détail dans les exemples suivants.

### Exemple 1 : synthèse de l'acide (1R,2R) 3-oxo-2-[(2Z)-2-pentenyl]- cyclopentaneacétique (+/-) de formule :

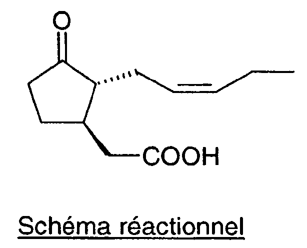

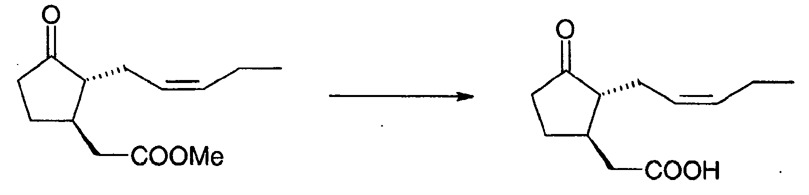

Dans un tricol de 250 ml muni d'un réfrigérant, d'un thermomètre et d'une agitation magnétique, on dissout 15 g (66,9 mmol) de (+/-) jasmonate de méthyle dans 150 ml d'acétone. On additionne lentement 10 ml de solution aqueuse de soude (5,35 g, 133,7 mmol). Le mélange est agité pendant 5 heures à température ambiante. L'acétone est alors évaporée sous vide puis la phase aqueuse résiduelle est lavée par de l'acétate d'éthyle (2 x 30 ml). La phase aqueuse est acidifiée par de l'acide chlorhydrique jusqu'à pH=2, puis extraite par du dichlorométhane (3 x 30 ml).
La phase organique est séchée sur sulfate de sodium, filtrée sur papier filtre puis concentrée. L'huile marron clair obtenue est séchée sous vide.
On obtient 13,6 g d'acide jasmonique (+/-) soit un rendement de 97%.
Le spectre RMN ¹H et le spectre de masse (ionisation négative) sont conformes à la structure attendue.

### Exemple 2 : synthèse du 3-(2-hydroxyethyl)-2-(2Z)-2-pentenyl-(2R,3R)-cyclopentanone de formule :

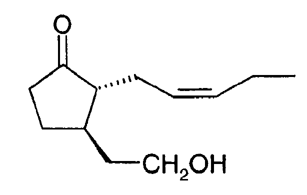

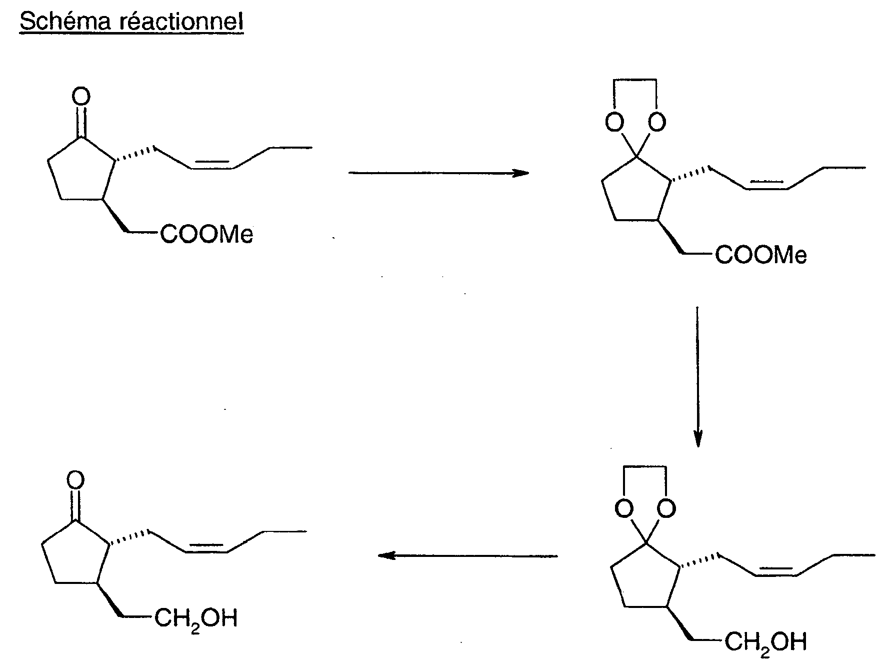

### 1/ étape 1

Dans un tricol de 250 ml muni d'un Dean Stark, d'un thermomètre ainsi que d'une agitation magnétique, on dissout 10 g de jasmonate de méthyle (44,6 mmol) dans 100 ml de toluène. On ajoute 20 g d'éthylène glycol (322,2 mmol) puis 2, 51 g de tosylate de pyridinium (10 mmol). Le mélange est agité au reflux pendant 16 heures en distillant l'eau formée à l'aide du Dean Stark. Le milieu réactionnel est alors concentré à sec. Le résidu est repris par 200 ml d'éther de méthyle et de tertiobutyle puis successivement lavé par une solution aqueuse de NaHCO₃ et par de l'eau salée. La phase organique est séchée sur sulfate de sodium, filtrée sur papier filtre puis concentrée.
On obtient 11, 5 g d'une huile qui est séchée sous vide (rendement : 96%).
Le spectre RMN ¹H est conforme à la structure attendue.

### 2/ étape 2

Dans un tricol de 250 ml muni d'un réfrigérant, d'un thermomètre ainsi que d'une agitation magnétique, on dissout 10 g du composé obtenu ci-dessus (37,3 mmol) dans 60 ml de tetrahydrofurane. On ajoute 2,85 g d'hydrure mixte de lithium et d'aluminium (75 mmol). Le mélange est agité pendant 4 heures à 30°C. Une fois la réaction terminée, on ajoute lentement 80 ml d'eau. Le précipité formé est filtré. Le filtrat est extrait par de l'acétate d'éthyle (3 x 60 ml). La phase organique est séchée sur sulfate de sodium, filtrée sur papier filtre puis concentrée. L'huile obtenue est purifiée par chromatographie sur gel de silice (éluant : éther de méthyle et tertiobutyle / méthyle cyclohexane).
On obtient 6,5 g d'une huile qui est séchée sous vide (rendement : 73%).
Le spectre RMN ¹H est conforme à la structure attendue.

### 3/ étape 3

Dans un tricol de 250 ml muni d'un réfrigérant, d'un thermomètre ainsi que d'une agitation magnétique, on place 5 g du composé obtenu à l'étape 2 ci-dessus (20,8 mmol) dans 20 ml de tétrahydrofurane. On ajoute 15 ml d'une solution d'acide chlorhydrique 2M et le milieu agité à température ambiante pendant 3 heures. Une fois la réaction terminée, le milieu réactionnel est neutralisé par une solution de NaHCO₃ puis extrait par de l'acétate d'éthyle (3 x 60 ml). La phase organique est séchée sur sulfate de sodium, filtrée sur papier filtre puis concentrée. L'huile obtenue est purifiée par chromatographie sur gel de silice (éluant : éther de méthyle et tertiobutyle / méthyle cyclohexane).
On obtient 3,8 g d'une huile qui est séchée sous vide (rendement : 93%).
Le spectre RMN ¹H est conforme à la structure attendue.

### Exemple 3 : tests d'activité

On étudie le pouvoir kératolytique de plusieurs composés selon l'invention. Ce test consiste en un comptage de cornéocytes libérés après incubation des lots de stratum corneum isolé en présence des composés testés.
On utilise du stratum corneum isolé par trypsine/chaleur à partir de plasties de chirurgie. On utilise plusieurs échantillons de stratum corneum différents. Des disques de 4 mm de diamètre sont coupés à l'emporte pièce et disposés au fond d'une boite de 96 puits.

### Test 1

On prépare une solution à 1% en poids de composé de l'exemple 1, ou d'acide (1 R,2R)-3-oxo-2-(2Z)-pentyl-cyclopentaneacétique (comparatif), dans un tampon PBS supplémenté à 0,1% en Triton X100. Le pH de la solution est réajusté à 7,4.
On ajoute dans chaque puits 50 microlitres de solution à tester ou de solution témoin (tampon PBS supplémenté à 0,1% en TritonX100). On laisse incuber à 37°C sous agitation pendant 24 heures.
On prélève alors 10 microlitres de solution, que l'on dispose en cellule de Mallassez. Les cornéocytes libérés sont comptés sous microscope.
On obtient les résultats suivants, exprimés en nombre de cornéocytes libérés par microlitre, moyennés pour trois essais. Les fragments de cornéocytes ne sont pas comptés.

| | Moyenne (3 essais par échantillon, 3 échantillons différents) |
|---|---|
| Exemple 1 | 15 ± 5 |
| Comparatif | 12 ± 4 |
| Témoin | 9 ± 3 |

Le nombre de cornéocytes libérés après incubation du stratum corneum isolé avec le composé selon l'invention est très supérieur au nombre libéré en présence du tampon seul, ou au nombre libéré en présence du comparatif (composé sans insaturation).

### Test 2

On prépare une solution à 1% en poids de composé de l'exemple 1 ou d'acide salicylique dans un tampon PBS supplémenté à 0,1% en Triton X100. Le pH des solutions est réajusté à 7,4.
On ajoute dans chaque puits 50 microlitres de solution à tester ou de solution témoin (tampon PBS supplémenté à 0,1% en TritonX100). On laisse incuber à 37°C sous agitation pendant 24 heures.
On prélève alors 10 microlitres de solution, que l'on dispose en cellule de Mallassez. Les cornéocytes libérés sont comptés sous microscope.
On obtient les résultats suivants, exprimés en nombre de cornéocytes libérés par microlitre. Les fragments de cornéocytes ne sont pas comptés.

| | Echantillon 1 * (3 essais) | Echantillon 2 * (3 essais) | Moyenne |
|---|---|---|---|
| Exemple 1 | 80 ± 4 | 64 ± 11 | 72 ± 7 |
| Acide salicylique | 33 ± 20 | 21 ± 8 | 27 ± 14 |
| Témoin | 29 ± 5 | 13 ± 4 | 21 ± 4 |

| | | | |
|---|---|---|---|
| * moyenne sur trois essais | | | |

### Exemple 4

On prépare une émulsion comprenant (% en poids):
- composé de l'exemple 1 1%
- isostéarate de propylène glycol 13%
- polyéthylène glycol (8 OE) 5%
- propylène glycol 3%
- pentylène glycol 3%
- stéarate de glycéryle et stéarate de polyéthylène glycol (100 OE) 5%
- mono-stéarate de sorbitane oxyéthyléné (20 OE) 0,5%
- alcool cétylique oxyéthyléné (20 OE) oxypropyléné (5 OP) 1%
- gélifiant 0,5%
- benzoates d'alkyle en C₁₂₋₁₅ 4%
- éthanol 3%
- hydroxyde de sodium 0,12%
- conservateurs qs
- eau qsp 100%

## Revendications

1. Utilisation cosmétique d'un composé de formule (I) : 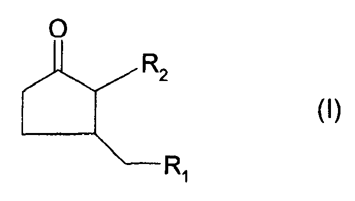 dans laquelle :
- R₁ est un radical choisi parmi -COOR', avec R' désignant un atome d'hydrogène ou un radical hydrocarboné, linéaire, ramifié ou cyclique, saturé ou insaturé, ayant 1 à 18 atomes de carbone,
- R₂ est un radical hydrocarboné, linéaire, ramifié ou cyclique, comportant au moins une insaturation, ayant 2 à 18 atomes de carbone,
et sels correspondants,
pour favoriser la desquamation de la peau et/ou stimuler le renouvellement épidermique.

2. Utilisation selon la revendication précédente, dans laquelle le radical R₁ est choisi parmi les radicaux -COOH, -COOCH₃, -COOC₂H₅, -COOC₃H₇.

3. Utilisation selon l'une des revendications précédentes, dans laquelle le radical R₁ est -COOH.

4. Utilisation selon l'une des revendications précédentes, dans laquelle le radical R₂ est un radical hydrocarboné, linéaire, ramifié ou cyclique, comportant une double insaturation, ayant 2 à 18 atomes de carbone, notamment 3 à 12 atomes de carbone, et en particulier 3 à 8 atomes de carbone.

5. Utilisation selon l'une des revendications précédentes, dans laquelle le radical R₂ représente un radical hydrocarboné linéaire, comportant une seule double insaturation, ayant 2 à 6 atomes de carbone, et notamment un radical -CH₂-CH=CH-C₂H₅.

6. Utilisation selon l'une des revendications précédentes, dans laquelle le composé de formule (I) est choisi parmi :
- l'acide 3-oxo-2-[(2Z)-2-pentenyl]- cyclopentaneacétique,
- le 3-oxo-2-[(2Z)-2-pentenyl]- cyclopentaneacétate de méthyle.

## Claims

1. Cosmetic use of a compound of formula (I): 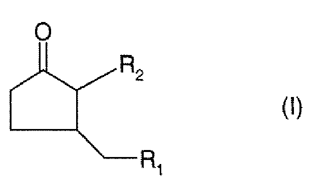 in which:
- R₁ is a radical selected from -COOR', where R' denotes a hydrogen atom or a saturated or unsaturated linear, branched or cyclic hydrocarbon radical having from 1 to 18 carbon atoms;
- R₂ is a linear, branched or cyclic hydrocarbon radical containing at least one unsaturation and having from 2 to 18 carbon atoms;
and corresponding salts;
to promote desquamation of the skin and/or to stimulate epidermal renewal.

2. Use according to the preceding claim, wherein the radical R₁ is selected from the radicals -COOH, -COOCH₃, -COOC₂H₅ and -COOC₃H₇.

3. Use according to either of the preceding claims, wherein the radical R₁ is -COOH.

4. Use according to one of the preceding claims, wherein the radical R₂ is a linear, branched or cyclic hydrocarbon radical containing a double unsaturation and having from 2 to 18 carbon atoms, in particular from 3 to 12 carbon atoms, and especially from 3 to 8 carbon atoms.

5. Use according to one of the preceding claims, wherein the radical R₂ represents a linear hydrocarbon radical containing a single double unsaturation and having from 2 to 6 carbon atoms, and in particular a radical -CH₂-CH=CH-C₂H₅.

6. Use according to one of the preceding claims, wherein the composition of formula (I) is selected from:
- 3-oxo-2-[(22)-2-pentenyl]cyclopentaneacetic acid, and
- methyl 3-oxo-2-[(22)-2-pentenyl]cyclopentaneacetate.

## Patentansprüche

1. Kosmetische Verwerdung einer Verbindung der Formel (I) : 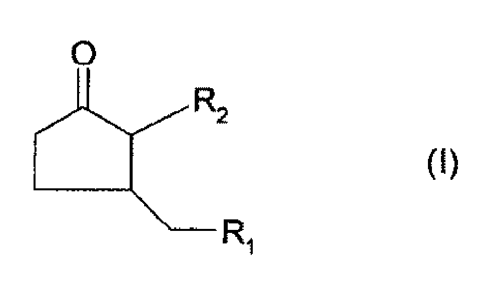 worin:
- R₁ für einen unter -COOR', wobei R' für ein Wasserstoffatom oder einen gesättigten oder ungesättigten, linearen, verzweigten oder cyclischen Kohlenwasserstoffrest mit 1 bis 18 Kohlenstoffatomen steht, ausgewählten Rest steht,
- R₂ für einen linearen, verzweigte oder cyclischen Kohlenwasserstoffrest mit mindestens einer Ungesättigtheit und 2 bis 18 Kohlenstoffatomen steht,
und entsprechenden Salzen
zur Förderung der Desquamation der Haut und/oder Stimulierung der Epidermiserneuerung.

2. Verwerdung nach dem vorhergehenden Anspruch, wobei der Rest R₁ unter den Resten -COOH, -COOCH₃, -COOC₂H₅ und -COOC₃H₇ ausgewählt ist.

3. Verwerdung nach einem der vorhergehenden Ansprüche, wobei der Rest R₁ für -COOH steht.

4. Verwendung nach einem der vorhergehenden Ansprüche, wobei der Rest R₂ für einen linearen, Verzweigten oder cyclischen Kohlenwasserstoffrest mit einer doppelten Ungesättigtheit und 2 bis 18 Kohlenstoffatomen, insbesondere 3 bis 12 Kohlenstoffatomen und speziell 3 bis 8 Kohlenstoffatomen, steht.

5. Verwendung nach einem der vorhergehenden Ansprüche, wobei der Rest R₂ für einen linearen Kohlenwasserstoffrest mit einer einzigen doppelten Ungesättigtheit und 2 bis 6 Kohlenstoffatomen und insbesondere einen -CH₂-CH=CH-C₂H₅-Rest steht.

6. Verwerdung nach einem der vorhergehenden Ansprüche, wobei die Verbindung der Formel (I) unter
- 3-Oxo-2-[(22)-2-pentenyl]cyclopentanessigsäure und
- 3-Oxo-2-[(22)-2-pentenyl]cyclopentanessigsäure-methylester
ausgewählt ist.
